# EUROPEAN PATENT APPLICATION

(11) **EP 4 242 292 A1**
(43) Date of publication of application: **13.09.2023**
(21) Application number: 21889449.1
(22) Date of filing: 26.10.2021
(51) Int. Cl.: C12M 3/00, C12M 1/32, C12M 1/00, C12N 5/00, C12N 5/071, C12N 5/0775

(54) **METHOD FOR PRODUCING EXTRACELLULAR VESICLES DERIVED FROM THREE-DIMENSIONAL SPHEROID-TYPE CELL AGGREGATE**

(30) Priority: 05.11.2020 KR 20200146489
(71) Applicant: S&E Bio Co., Ltd., Seoul 06351 (KR)
(72) Inventor: KIM, Eun Hee, Seoul 06608 (KR); SHIN, Eun Kyoung, Seoul 07611 (KR); SUNG, Ji Hee, Seoul 08716 (KR)
(74) Representative: Potter Clarkson
(86) International application number: PCT/KR2021/015122
(87) International publication number: WO 2022/097984

(57) **Abstract**

The present invention relates to: a method in which extracellular vesicles with improved therapeutic efficacy are produced from a three-dimensional spheroid-type cell aggregate produced using a microwell having a particular structure; and extracellular vesicles produced by the production method. When the method for producing extracellular vesicles, according to the present invention, is used, extracellular vesicles having enhanced efficacy can be mass-produced, and the produced extracellular vesicles include higher levels of various efficiency factors than existing extracellular vesicles and exhibit uniform characteristics without difference between donors, and thus can be used for various purposes in the medical industry.

## Description

### [Technical Field]

The present invention relates to a method for producing a three-dimensional spheroid-type cell aggregate using a microwell having a particular structure and producing extracellular vesicles with improved therapeutic efficacy therefrom, and extracellular vesicles produced by the production method.

### [Background Art]

Treatments using stem cells, particularly mesenchymal stem cells (MSC), and positive clinical results have been reported for various diseases. In the therapeutic efficacy of stem cell therapies, it is reported that the regeneration and revascularization ability of surrounding skin cells have been enhanced due to a paracrine effect of most of the stem cells. Extracellular vesicles (EVs) are known as a main efficacy factor for the paracrine effect. Recently, substances resulting from the secretion of extracellular vesicles secreted from stem cells have attracted attention for mediating various stem cell therapeutic effects. The extracellular vesicles have the sizes of 30 to 5,000 nm and are classified into exosomes, microvesicles, and apoptotic bodies by size, and are also called ectosomes, prostatosomes, etc. depending on a type and an origin.

However, mass production, obtaining methods, and the like for using extracellular vesicles derived from stem cells have not yet been established. In the case of conventional methods for producing two-dimensional extracellular vesicles, since it is difficult to mass-produce the extracellular vesicles due to using animal serum (xenogeneic serum) or the limited secretion of extracellular vesicles from stem cells, there is a need for a new production method capable of mass-producing extracellular vesicles that can be applied clinically.

### [Disclosure]

### [Technical Problem]

Therefore, the present inventors studied a method capable of shortening a culture time while enabling mass production to produce a three-dimensional spheroid-type cell aggregate or extracellular vesicles derived therefrom. As a result, the present inventors confirmed that extracellular vesicles with an improved therapeutic efficacy substance were produced by producing a three-dimensional spheroid-type cell aggregate (3D-static-spheroid) through static culture using a microwell and producing extracellular vesicles using the same, and then completed the present invention.

Accordingly, an object of the present invention is to provide a method for producing extracellular vesicles with an improved efficacy factor level by producing a three-dimensional spheroid-type cell aggregate through a microwell and extracellular vesicles produced by the production method.

### [Technical Solution]

One aspect of the present invention provides a method for producing extracellular vesicles derived from a three-dimensional spheroid-type cell aggregate including (a) preparing a three-dimensional spheroid-type cell aggregate by 3 dimensionally (3D)-culturing mesenchymal stem cells in a microwell with a diameter of 200 to 800 µm and a depth of 100 to 1000 µm; and (b) isolating extracellular vesicles from the three-dimensional spheroid-type cell aggregate.

Another aspect of the present invention provides extracellular vesicles produced by the production method.

Yet another aspect of the present invention provides a composition for use in producing extracellular vesicles including a three-dimensional spheroid-type cell aggregate produced by 3 dimensionally (3D)-culturing mesenchymal stem cells in a microwell with a diameter of 200 to 800 µm and a depth of 100 to 1000 µm.

Still another aspect of the present invention provides a method for reducing a donor variation of extracellular vesicles derived from a cell aggregate including (a) preparing a three-dimensional spheroid-type cell aggregate by 3 dimensionally (3D)-culturing stem cells in a microwell with a diameter of 200 to 800 µm and a depth of 100 to 1000 µm; and (b) isolating extracellular vesicles from the three-dimensional spheroid-type cell aggregate.

Still yet another aspect of the present invention provides a method for producing extracellular vesicles derived from a cell aggregate with a reduced donor variation including (a) preparing a three-dimensional spheroid-type cell aggregate by 3 dimensionally (3D)-culturing stem cells in a microwell with a diameter of 200 to 800 µm and a depth of 100 to 1000 µm; and (b) isolating extracellular vesicles from the three-dimensional spheroid-type cell aggregate.

### [Advantageous Effects]

According to the present invention, when the method for producing the extracellular vesicles is used, it is possible to mass-produce extracellular vesicles with enhanced efficacy, and the produced extracellular vesicles include various efficacy factors at higher levels than existing extracellular vesicles and exhibit uniform characteristics without difference between donors, and thus can be used for various purposes in the medical industry.

### [Description of Drawings]

FIG. 1 is a diagram illustrating a process of producing a 3D spheroid-type cell aggregate and isolating extracellular vesicles therefrom.
FIG. 2A is a diagram of observing a microwell containing a 3D-dynamic-PEG spheroid culture medium.
FIG. 2B is a diagram of observing a microwell containing the 3D-static spheroid culture medium.
FIG. 2C is a diagram illustrating changes in aggregate area according to culture periods of 3D-dynamic-PEG spheroid and 3D-static-spheroid produced using a microwell.
FIG. 3 is a diagram comparing size distributions of 3D-static-spheroid and 3D-dynamic-PEG spheroid.
FIG. 4 is a diagram of observing the shape of 3D-static-spheroid EVs with an electron microscope.
FIG. 5 is a diagram illustrating results of nanoparticle tracking analysis (NTA) to confirm the concentration and size distribution of 3D-static-spheroid EVs.
FIG. 6 is a diagram illustrating results of ELISA and Western blot to confirm expression markers of 3D-static-spheroid EVs.
FIG. 7 is a diagram comparing EV productions per cell derived from 3D-static-spheroid EVs, 3D-dynamic-PEG spheroid EVs, and 2D-EVs.
FIG. 8 is a diagram illustrating miRNAs and proteins highly expressed in 3D-static-spheroid EVs compared to 2D-EVs.
FIG. 9 is a diagram illustrating miRNAs and proteins highly expressed in 3D-static-spheroid EVs compared to 3D-dynamic-PEG spheroid EVs.
FIG. 10 is a diagram illustrating results of confirming differences in expression levels of miRNAs related to donor variation, angiogenesis, and nerve regeneration in 2D cultured WJ-MSC, 3D cultured WJ-MSC, 2D-EV, and 3D-static-spheroid EV.
FIG. 11 is a diagram confirming changes in mRNA expression after treating 3D-static-spheroid EVs or 2D-EVs to human umbilical vascular endothelial cells (HUVECs) or primitive neural stem cells (pNSCs) as neural stem cells.
FIG. 12 is a diagram illustrating changes in infarct area according to a treatment concentration of 3D-static-spheroid EVs in a stroke rat model.
FIG. 13 is a diagram illustrating the measurement of neurological behavior recovery ability according to a treatment concentration of 3D-static-spheroid EVs by performing a ladder walking test for a stroke rat model.
FIG. 14 is a diagram of observing cells treated with CFSE-labeled 3D-static-spheroid EVs through confocal microscopy.
FIG. 15 is a diagram confirming that miR-27a-3p is delivered to cells by CFSE-labeled 3D-static-spheroid EVs.

### [Best Mode of the Invention]

The present invention provides a method for producing extracellular vesicles derived from a three-dimensional spheroid-type cell aggregate including (a) preparing a three-dimensional spheroid-type cell aggregate by 3-dimensionally culturing stem cells in a microwell with a diameter of 200 to 800 µm and a depth of 100 to 1000 µm; and (b) isolating extracellular vesicles from the three-dimensional spheroid-type cell aggregate.

In the present invention, the cells may be used without limitation as long as the cells are cells capable of isolating extracellular vesicles, and may be cells isolated from a natural organism. In addition, the cells may be derived from any type of animal or plant, including humans and non-human mammals, and may be various types of immune cells, tumor cells, and stem cells, and preferably, the stem cells may be mesenchymal stem cells, pluripotent stem cells, induced pluripotent stem cells, or embryonic stem cells.

In the present invention, the 3D culture means culturing in a three-dimensional arrangement *in vitro,* and unlike 2D culture, the 3D culture may grow in all directions *in vitro* and create an environment more similar to an *in vivo* cell environment.

In the present invention, the 3D culture in step (a) may be performed by all 3D cell culture techniques known in the art to which the present invention pertains, and may be a cell culture using, for example, microwell array culture, porous microsphere culture, hanging drop culture, low attachment plate culture, membrane-based cell-detachment culture, thermal lifting culture, centrifugation culture, semisolid medium culture, and the like. Preferably, the 3D culture may be a dynamic culture or static culture, more preferably static culture. In the present invention, when the 3D culture in step (a) is the static culture, it makes it easier to culture without requiring devices necessary for shaking culture, and mass-culture is enabled in the good manufacturing practices (GMP) manufacturing place.

In the present invention, the 3D culture in step (a) may be performed for 1 to 10 days, preferably 2 to 4 days. Particularly, in the present invention, when the culture in step (a) is performed for 2 to 4 days, the viability of cells present in the three-dimensional spheroid-type cell aggregate is maintained at a high level, and compared to a conventional process of producing a three-dimensional spheroid-type cell aggregate, the culture time is relatively short, so that it is possible to rapidly produce a three-dimensional spheroid-type cell aggregate and extracellular vesicles derived therefrom.

In the present invention, the 3D culture in step (a) may be performed by dispensing mesenchymal stem cells in a microwell at a density of 100 to 1000 cells/well, preferably a density of 200 to 600 cells/well.

In the present invention, the isolating of the extracellular vesicles in step (b) may be performed by physical isolation or chemical isolation. The physical isolation may be extrusion of a sample containing cells or a cell aggregate, and the chemical isolation may be treatment with a chemical substance capable of isolating extracellular vesicles from cells or a cell aggregate. For example, the isolating of the present invention may be performed by using a method selected from the group consisting of sonication, cell lysis, homogenization, freeze-thawing, electroporation, chemical treatment, mechanical degradation, and treatment of physical stimuli externally applied to cells. The isolating may be performed by, as an ion or affinity chromatography method, a physiochemical method of isolation through binding of ions to specific biomarkers or an immunoaffinity EV capture, exosome purification reagent, ExoCAS-2 reagent or method which destroys cells and binds and isolates only extracellular vesicles through polymers and reagents, and preferably may be performed by a tangential flow filtration (TFF) method, but is not limited thereto.

In the present invention, MicroRNA is a type of RNA and is named by attaching "mir" in the front and adding "-" and a number at the end. At this time, the numbers often indicate the order to be named, and for example, mir-123 was named before mir-156 and is expected to have been identified earlier. "mir-" indicates pre-microRNA, and capitalized "miR" refers to mature microRNA. Except for one or two sequences, microRNAs with almost identical sequences are named by adding small letters. For example, miR-121a and miR-121b were generated from respective precursors mir-121a and mir-121b, and their sequences are very similar. Mature microRNAs are identical, but pre-microRNAs located at different sites on a genome are named by adding "-" and a number. For example, the pre-microRNAs mir-121-1 and mir-121-2 become the same mature microRNA (miR-121), but are located at different sites on the genome. Species-specific names of microRNAs are indicated in the front. For example, hsa-miR-123 is Homo sapiens microRNA, and oar-miR-123 is Ovis aries microRNA. 'v' means viral (miRNA encoded by a viral genome) and 'd' means Drosophila microRNA. When two mature microRNAs are derived from different arms (3' arm or 5' arm) of the same pre-microRNA, the mature microRNAs are named by adding `-3p' or '-5p' to the end. (In the past, the classification was indicated as 's' (sense) and 'as' (antisense)). miR-142-3p is derived from the 3' arm, and miR-142-5p is derived from the 5' arm. The nomenclature of MicroRNA generally follows the above criteria, but there are exceptions.

In the present invention, the extracellular vesicles may highly express various substances exhibiting therapeutic efficacy compared to known extracellular vesicles, and the various substances exhibiting the therapeutic efficacy may be substances exhibiting effects on angiogenesis, neurogenesis, cytoprotection, immunomodulation, rejuvenation, vascular/BBB permeation stabilization, anticancer effect, or targeting. Preferably, the extracellular vesicles may highly express at least one selected from the group consisting of miR-146a, miR-27a, miR-132, miR-184, miR-210, and miR-301b, and/or at least one selected from the group consisting of integrin 1/2 and Vascular Endothelial Growth Factor Receptor 2 (VEGF/R2) compared to extracellular vesicles derived from a spheroid-type cell aggregate obtained by 3D dynamic culture of mesenchymal stem cells. In addition, the extracellular vesicles may highly express at least one selected from the group consisting of miR-27a, miR-146a, and miR-146b and at least one selected from the group consisting of integrin 1/2 and Vascular Endothelial Growth Factor Receptor 2 (VEGF/R2) compared to extracellular vesicles derived from 2D-cultured mesenchymal stem cells. More preferably, the extracellular vesicles of the present invention may highly express miR-146a, miR-27a, miR-132, miR-184, miR-210, and miR-301b; and/or integrin 1/2 and VEGF/R2 compared to extracellular vesicles derived from a spheroid-type cell aggregate obtained by 3D dynamic culture of mesenchymal stem cells, and may highly express miR-27a, miR-146a and miR-146b; and/or integrin 1/2 and VEGF/R2 compared to extracellular vesicles derived from 2D-cultured mesenchymal stem cells.

In addition, the extracellular vesicles may be incorporated into cells and internalized upon treatment with cells, and may be incorporated into cells and internalized. As a result, a clinically significant substance may be effectively delivered to cells to be highly expressed in cells.

In the present invention, the "extracellular vesicles derived from the spheroid-type cell aggregate obtained by 3D dynamic culture of mesenchymal stem cells" are any extracellular vesicles isolated from a spheroid-type cell aggregate obtained by 3D dynamic culture of mesenchymal stem cells without limitation, preferably extracellular vesicles disclosed in Registration Patent (application No. 10-2016-0053026, method for producing stem cell-derived extracellular vesicles).

In the present invention, the "extracellular vesicles derived from 2D-cultured mesenchymal stem cells" may be extracellular vesicles obtained by culturing stem cells according to a conventional 2D culture method and isolated from the stem cells by a conventional method of isolating extracellular vesicles without limitation. In an embodiment of the present invention, the conventional 2D culture method was evaluated by performing a method in which stem cells cultured for 3 days in a cell stack were washed with PBS, replaced with a serum-free medium, and further cultured for 2 days.

In the present invention, the three-dimensional spheroid-type cell aggregate may have an average diameter of 65 to 133 µm, preferably 99.20 ± 11.47 µm. More specifically, as a result of measuring the size distribution of 154 three-dimensional spheroid-type cell aggregates, the average diameter may be 99.20 µm and the coefficient of variance (CV) may be 11.6%. The dimensional spheroid-type cell aggregate of the present invention may have a higher kurtosis in size distribution than the "spheroid-type cell aggregate obtained by 3D dynamic culture of mesenchymal stem cells". Therefore, the size of the three-dimensional spheroid-type cell aggregate is smaller than that of the "spheroid-type cell aggregate obtained by 3D dynamic culture of mesenchymal stem cells", and may have a relatively uniform size distribution.

In the present invention, the microwell may be coated with at least one selected from the group consisting of TMSPMA (3-(Trimetoxysily) propylmethacrylate), HEA (Hydroxyethyl acrylate), GMA (Glycidyl methacrylate), EGDMA (diethyleneglycol dimethacrylate), THFA (Tetrahydrofurfuryl acrylate), HMAA (Hydroxymethul acrylamide), PEA (Phenyl epoxyacrylate), HOFHA (6-Hydroxy-2, 2,3,3,4,4,5,5-octafluoro), EOPT (Polyethoxylated(4)pentaerythritoltetraacrylate), HPA (Hydroxypropyl acrylate), BMA (Buthylmethacrlate), PETIA (Pentaerythritol triacrylate), HDDA (Hexan diol diacrylate), EGPEA (Ethyleneglycol phenyletheracrylate), BM (Benzylmethacrylate), HPPA (Hydroxyphenoxypropyl acrylate), BHPEA (2-(4-Benzoyl-3-hydroxyphenoxy)ethylacrylate), HEMA (Hydroxyethyl methacrylate), HPMA (N-(2-Hydroxypropyl) methacrylamide) and MPC (2-Methacryloyloxyethyl Phosphorylcholine Polymer), preferably may be coated with MPC (2-Methacryloyloxyethyl Phosphorylcholine Polymer), but is not limited thereto.

The microwell of the present invention may have a structure with a diameter of 200 to 800 µm and a depth of 100 to 1000 µm, and the mesenchymal stem cells cultured by the structure may maintain a high survival rate even after the culture time has elapsed.

When the extracellular vesicles are produced by the "method for producing the extracellular vesicles derived from the three-dimensional spheroid-type cell aggregate" of the present invention, in addition to the manufacturing advantages of static culture, extracellular vesicles with an improved therapeutic efficacy substance may be rapidly and efficiently mass-produced. In particular, the method for producing the extracellular vesicles of the present invention is suitable for GMP applications.

In addition, the present invention provides extracellular vesicles produced by the production method.

In the present invention, the extracellular vesicles may highly express the therapeutic efficacy substance compared to known extracellular vesicles. Preferably, the extracellular vesicles may highly express at least one selected from the group consisting of miR-146a, miR-27a, miR-132, miR-184, miR-210, and miR-301b, and/or at least one selected from the group consisting of integrin 1/2, and Vascular Endothelial Growth Factor Receptor 2 (VEGF/R2) compared to extracellular vesicles derived from a spheroid-type cell aggregate obtained by 3D dynamic culture of mesenchymal stem cells. In addition, the extracellular vesicles may highly express at least one selected from the group consisting of miR-27a, miR-146a, and miR-146b and at least one selected from the group consisting of integrin 1/2 and Vascular Endothelial Growth Factor Receptor 2 (VEGF/R2) compared to extracellular vesicles derived from 2D-cultured mesenchymal stem cells. More preferably, the extracellular vesicles of the present invention may highly express miR-146a, miR-27a, miR-132, miR-184, miR-210, and miR-301b; and/or integrin 1/2 and VEGF/R2 compared to extracellular vesicles derived from a spheroid-type cell aggregate obtained by 3D dynamic culture of mesenchymal stem cells, and may highly express miR-27a, miR-146a and miR-146b; and/or integrin 1/2 and VEGF/R2 compared to extracellular vesicles derived from 2D-cultured mesenchymal stem cells.

In the present invention, the extracellular vesicles may have an average particle diameter of 172.5 to 192.5 nm and a mode diameter of 96.1 to 116.1 nm.

In addition, the extracellular vesicles derived from the three-dimensional spheroid-type cell aggregate produced by the production method of the present invention are particularly reduced in donor variation.

In the present invention, the extracellular vesicles may reduce a brain infarct area and induce neurological behavioral recovery when treated to a subject with brain infarct. Preferably, the extracellular vesicles may exhibit an effect of reducing the brain infarct area and inducing neurological behavior recovery in a small amount compared to extracellular vesicles produced by a conventionally known method.

In addition, the present invention provides a composition for use in producing extracellular vesicles including a three-dimensional spheroid-type cell aggregate produced by 3D culture of mesenchymal stem cells in a microwell with a diameter of 200 to 800 µm and a depth of 100 to 1000 µm.

In the present invention, the composition may include without limitation any components necessary for producing the extracellular vesicles.

In addition, the present invention provides a method for reducing a donor variation of extracellular vesicles derived from a cell aggregate including (a) preparing a three-dimensional spheroid-type cell aggregate by 3-dimensionally (3D) culturing stem cells in a microwell with a diameter of 200 to 800 µm and a depth of 100 to 1000 µm; and (b) isolating extracellular vesicles from the three-dimensional spheroid-type cell aggregate.

In addition, the present invention provides a method for producing extracellular vesicles derived from a cell aggregate with a reduced donor variation including (a) preparing a three-dimensional spheroid-type cell aggregate by 3-dimensionally (3D) culturing stem cells in a microwell with a diameter of 200 to 800 µm and a depth of 100 to 1000 µm; and (b) isolating extracellular vesicles from the three-dimensional spheroid-type cell aggregate.

According to the method of the present invention, it is possible to produce extracellular vesicles having uniform characteristics by reducing a difference in miRNA composition according to a donor between the obtained cell aggregate-derived extracellular vesicles. More specifically, in the present invention, the reduction of the donor variation is to reduce a difference in which the miRNA composition produced varies according to a donor to exhibit a consistent miRNA profile regardless of the donor. Much more specifically, compared to 2D-EVs, miR-27a-3p, miR-146a-5p, miR-210, and miR-132, which are miRNAs capable of exhibiting angiogenic or nerve regenerative effects, are more uniformly included, or miR-199a, miR-125b, miR-26a, let-7, miR-125a, miR-181b, and miR-92a, which are miRNAs related to angiogenesis or nerve regeneration, may be expressed at a uniform level regardless of the origin of each donor.

Duplicated contents are omitted in consideration of the complexity of the present specification, and terms not defined otherwise in the present specification have the meanings commonly used in the art to which the present invention pertains.

Hereinafter, the present invention will be described in detail by Examples. However, the following Examples are just illustrative of the present invention, and the contents of the present invention are not limited to the following Examples.

### [Modes of the Invention]

### Western blot

Cells and extracellular vesicles were dissolved in a radioimmunoprecipitation assay (RIPA) buffer (25 mM Tris-HCl, pH 7.6, 150 mM NaCl, 0.5% triton X-100, 1% Na-deoxycholate, 0.1% sodium dodecyl sulfate (SDS) and protease inhibitor). A total of 20 µg of proteins was isolated by SDS-polyacrylamide gel electrophoresis and transferred to a nitrocellulose membrane (Bio-Rad, Hercules, CA, USA). Thereafter, the nitrocellulose membrane was cultured overnight at 4°C together with a primary antibody against histone H2A.Z, histone H3, lamin A/C, flotillin-1 (1 : 1,000, Cell signaling technology, Beverly, MA, USA) or calreticulin (1 : 1,000, ThermoFisher Scientific, Inc., Rockford, IL, USA). After washing with Tris-buffered saline-Tween 20, the nitrocellulose membrane was cultured for 2 hours together with a horseradish peroxidase (HRP)-conjugated secondary antibody (1 : 1,000, anti-rabbit, Cell Signaling Technology, Beverly, MA, USA). The proteins were detected using a chemiluminescent substrate from ThermoFisher Scientific, Inc. (Waltham, MA, USA). Labeled proteins were visualized through an X-ray film (Agfa, Mortsel, Belgium).

### ELISA

ELISA was performed with a commercial kit according to the manual of an individual manufacturer. The following ELISA kits were used: gentamicin (5111GEN, EuroProxima, Arnhem, Nederland), bovine albumin (8100, Alpha Diagnostic, San Antonio, TX, USA), Hsp70 (Abcam, Cambridge, UK), CD63, CD9 and CD81 (System Biosciences, Palo Alto, CA, USA), histone H2A.Z. (Mybiosource, San Diego, CA, USA), calreticulin (Mybiosource) and cytochrome C (ThermoFisher Scientific, Inc.). All the kits included standard proteins. Therefore, the amounts of proteins and extracellular vesicles were determined based on a standard curve of each kit.

### qPCR

RNA was extracted from EVs using Trizol^{™} according to the instructions of the manufacturer and RNA was quantified by a nanodrop. RNA was made into cDNA through a reverse transcription (RT) process, and real-time PCR was performed according to the manual of the manufacturer using Taqman probes suitable for each miRNA and mRNA.

### EV labeling and uptake by cells

Purified EVs were stained with CFSE (5-(and-6)-Carboxyfluorescein Diacetate, Succinimidyl Ester) labeling dye (c-1157, Invitrogen) according to the manufacturer's instructions. Excess dye was removed by ultracentrifugation at 100.000 g for 1 hour. A human NSC cell line (ReNcells^{®}) was treated with the labeled EVs (0.4 µg/ml) and cultured for 24 hours. After treatment, cells were washed twice with PBS and stained using a conventional immunocytochemistry protocol. The cells were cultured overnight at 4°C with a mouse anti-SMA (1 : 100, Sigma Aldrich) antibody. Thereafter, the cells were washed with PBS and cultured with a secondary antibody, DyLight-labeled anti-mouse IgG (1 : 200, 594 nm, Abcam) antibody. The cells were nuclear-stained using Vectashield^{™} with 1.5 µg/mL 4'-6' diamidino-2-phenylindole (DAPI) (Vector Laboratories), and then the cells were imaged using a confocal microscope (LSM 700, Carl Zeiss, Germany).

### Example 1. Isolation of extracellular vesicles through 3D culture of mesenchymal stem cells

### 1.1 Preparation of mesenchymal stem cells

Human umbilical cord-derived mesenchymal stem cells (Wharton's Jelly-derived mesenchymal stem cells) (hereinafter referred to as WJ-MSC, Samsung medical center, Seoul, Korea) at passage 5 stage were received and cultured in a 37°C, 5% CO₂ incubator. A growth medium was used with an α-modified eagle's medium (α-MEM, GIBCO, NY, USA) containing 10% fetal bovine serum (FBS) (GIBCO, NY, USA) and 50 µg/mL gentamicin (GIBCO, NY, USA). WJ-MSCs at passage 6 stage were used to prepare a 3D spheroid-type cell aggregate.

### 1.2 Production of 3D spheroid-type cell aggregate culture medium

The WJ-MSCs prepared in Example 1.1 were washed with PBS, treated with trypsin (TrypLE^{™} Express, GIBCO, NY, USA) and reacted in a CO₂ incubator for 5 minutes. Thereafter, a new serum-free medium was added to neutralize trypsin and the cells were recovered to obtain a cell pellet using a centrifuge. Then, a new serum-free medium was added to prepare a cell suspension, and the cells were counted. After cell counting, 60 ml of the cell suspension was uniformly dispensed in a microarray including microwells coated with 2-methacryloyloxyethyl phosphorylcholine polymer (MPC) with a diameter and depth of 500 µm × 200 µm, respectively, to be a density of 400 cells/well, and maintained in a static state to induce the spontaneous formation of spheroid-type cell aggregates, and cultured in a CO₂ incubator at 37°C for a total of 4 days to prepare a 3D spheroid-type cell aggregate culture medium (hereinafter, referred to as a 3D-static-spheroid culture medium).

### 1.3 Isolation of extracellular vesicles derived from 3D spheroid-type cell aggregate

The 3D-static-spheroid culture medium prepared in Example 1.2 was collected, centrifuged at 2,500 g for 10 minutes, and filtered with a 0.22 µm syringe filter to remove cell debris. Thereafter, the 3D-static-spheroid culture medium passed through 300 kDa of a hollow fiber membrane (Pall, NY, USA) using a tangential flow filtration (TFF) system to remove proteins, and extracellular vesicles were first isolated and purified once more with physiological saline to obtain high-purity 3D-static-spheroid-derived extracellular vesicles (hereinafter, 3D-static-spheroid EVs) of the present invention.

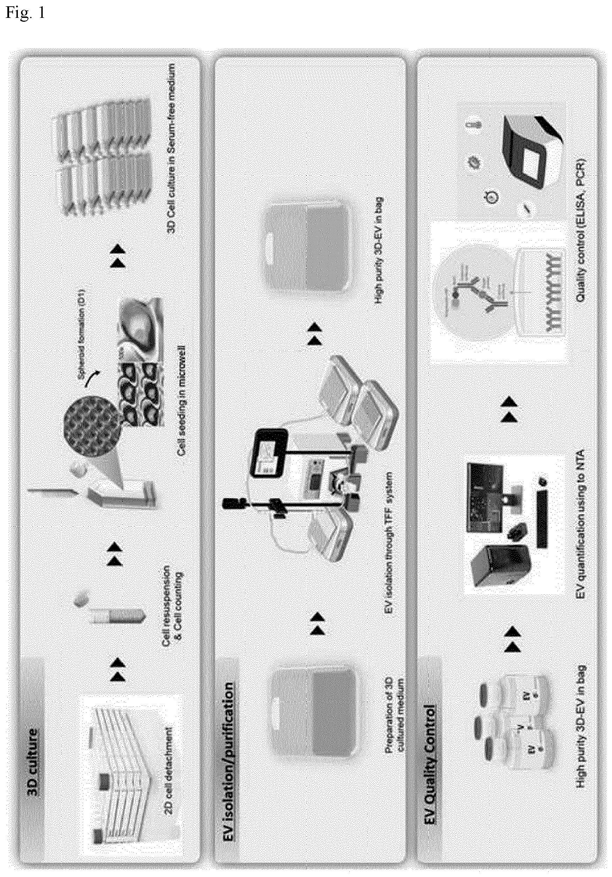

The processes of Examples 1.1 to 1.3 were schematically illustrated in FIG. 1.

### Example 2. Analysis of characteristics of 3D spheroid-type cell aggregate

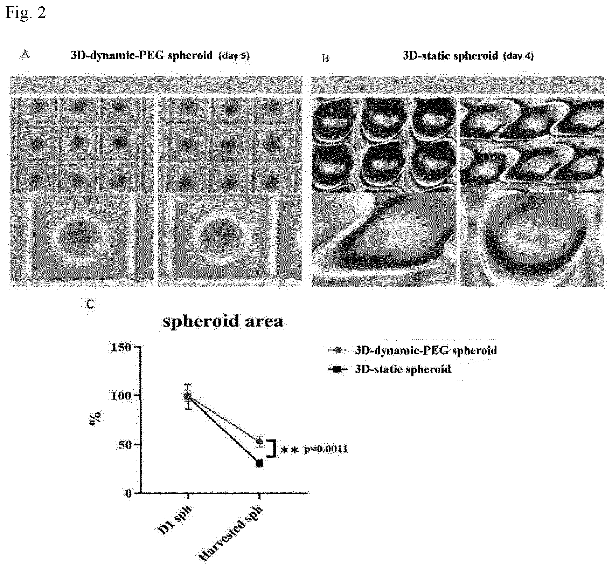

The characteristics of the 3D-spheroid-type cell aggregate (hereinafter, referred to as 3D-static-spheroid) present in the 3D-static-spheroid culture medium prepared in Example 1.2 were analyzed. As an experimental group, the 3D-static-spheroid culture medium prepared in Example 1.2 was used, and as a comparative group, a 3D mesenchymal stem cell spheroid-type cell aggregate (hereinafter referred to as 3D-dynamic-PEG spheroid) culture medium prepared by culturing mesenchymal stem cells for 5 days was used according to a dynamic 3D cell culture method disclosed in Registered Patent (Application No. 10-2016-0053026, method for producing stem cell-derived extracellular vesicles). With an optical microscope, microwells containing each culture medium were observed, which were illustrated in FIGS. 2A and 2B, and changes in spheroid-type cell aggregate area after culture compared to the initial culture were illustrated in FIG. 2C.

As illustrated in FIG. 2, as a result of comparing the areas of the 3D-static-spheroid of the experimental group and the 3D-dynamic-PEG spheroid of the control group, the 3D-static-spheroid maintained the area of about 70% compared to the spheroid at the initial culture. However, cell debris occurred in the 3D-dynamic-PEG spheroid, and the area of the 3D-dynamic-PEG spheroid was reduced to about 50% compared to the area of the spheroid at the initial culture, and a difference in area reduction rate between the 3D-static-spheroid and the 3D-dynamic-PEG spheroid was statistically significant with p ≤ 0.5 (p = 0.019753).

### Example 3. Size analysis of 3D spheroid-type cell aggregate

The size distributions of the 3D-static-spheroid prepared in Example 1.2 and the 3D-dynamic-PEG spheroid prepared in Example 3 were measured, and based on the measured size distribution data, dispersion coefficient, skewness and kurtosis were analyzed.

The skewness was a parameter capable of determining the inclined direction and degree of the distribution from the trend of a median value, and indicated a distribution with a longer tail to the right as closer to 1, and indicated a distribution with a longer tail to the left as closer to -1. In the case of a normal distribution, the skewness was 0.

The kurtosis was a parameter that indicated the degree of sharpness of the data distribution, and if the value was positive, it meant that a relatively large number of data points were accumulated in the central part, and if the value was negative, it meant that relatively few data were accumulated in the central part. In the case of a normal distribution, the kurtosis was 0.

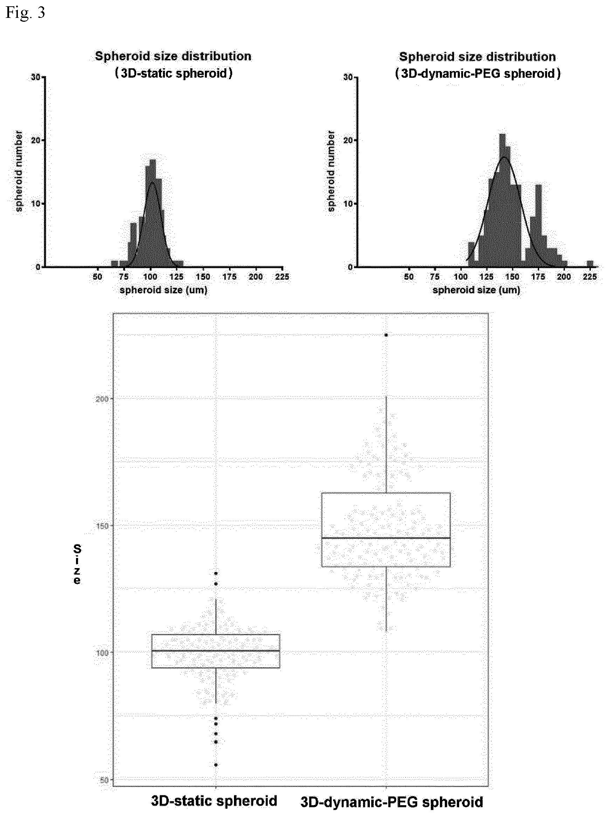

The measured size distribution data was illustrated in FIG. 3, and the results of analyzing the data was illustrated in Table 1.

**[Table 1]**

| | 3D-dynamic-PEG spheroid | 3D-static-spheroid |
|---|---|---|
| The number of spheroids | 154 | 154 |
| Size range | 108.4 - 225.0 um | 55.9 - 131.0 um |
| Size (mean ± SD) | 148.66 ± 20.89 um | 99.20 ± 11.47 um |
| Coefficient of variance | 14.1% | 11.6% |
| Skewness | 0.694 ± 0.195 | - 0.745 ± 0.195 |
| Kurtosis | 0.350 ± 0.389 | 1.799 ± 0.389 |

As illustrated in FIG. 3 and Table 1, it was confirmed that the average size of the 3D-static-spheroids was 99.20 um, and the coefficient of variance (CV) was 11.6%. In contrast, it was confirmed that the 3D-dynamic-PEG spheroids had an average size of 148.66 um and a coefficient of variance (CV) of 14.1%. To compare and test the measured coefficient of variance, as a result of performing a Feltz and Miller's (1996) asymptotic test, a p value was calculated as 0.01765604, and as a result of performing a Krishnamoorthy and Lee's (2014) modified signed-likelihood ratio test, the p value was calculated as 0.01853969. Accordingly, in both the tests, it was confirmed that the size distributions of 3D-static-spheroids and 3D-dynamic-PEG spheroids showed statistically significant different patterns. As a result of comparing the size distributions of the 3D-static-spheroids and the 3D-dynamic-PEG spheroids, it was confirmed that the kurtosis value of the size distribution of the 3D-static-spheroids was relatively large, and the size distribution of the 3D-static-spheroids of the present invention showed a tendency to be concentrated in the median values. From these results, when the 3D spheroids were produced by the method for producing the 3D-static-spheroids of the present invention, it was confirmed that it was possible to produce 3D spheroids having a relatively constant size.

### Example 4. Morphological analysis of extracellular vesicles derived from 3D spheroid-type cell aggregate

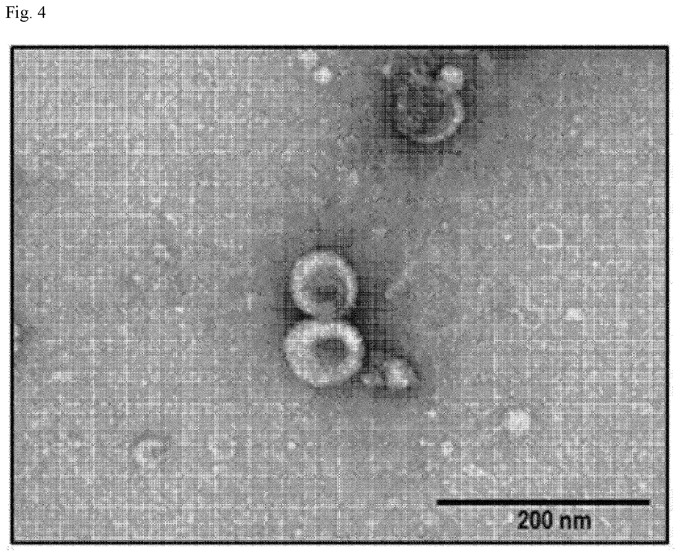

In order to observe the morphology of the 3D-static-spheroid EVs isolated in Example 1.3, the morphology was photographed using a transmission electron microscopy (TEM). Specifically, the 3D-static-spheroid EVs were fixed with 1% OsO₄ dissolved in a 0.1 M phosphate buffer (PB) for 2 hours. The EM grids were adsorbed onto the droplets of extracellular vesicles with a formvar side facing downward for 1 minute. Thereafter, the EM grids were blotted with filter paper and reacted with 2% uranyl acetate for 15 seconds. Excessive uranyl acetate was removed, and the EM grids were observed using a TEM (JEM-1011, JEOL, Japan), and the observed image was illustrated in FIG. 4.

As illustrated in FIG. 4, it was confirmed that the 3D-static-spheroid EV had a round shape, which was a typical shape of extracellular vesicles.

### Example 5. Concentration and size analysis of extracellular vesicles derived from 3D spheroid-type cell aggregate

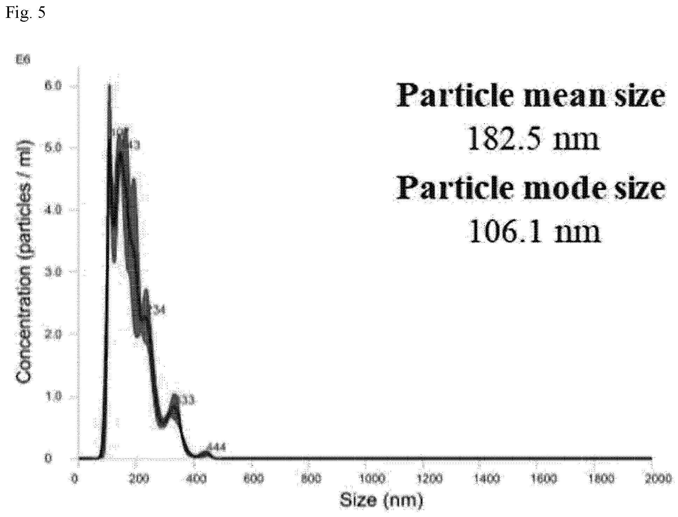

In order to confirm the concentration and size distribution of 3D-static-spheroid EVs isolated in Example 1.3, nanoparticle tracking analysis (NTA) was performed using NanoSight NS300 (Malvern, Worcestershire, UK). For optimal analysis, the 3D-static-spheroid EVs were pre-diluted in a vesicle-free phosphate buffer (PBS). The average size and concentration (particle/mL) were calculated by integrating three records, and the results were illustrated in FIG. 5.

As illustrated in FIG. 5, it was confirmed that the average particle diameter of the 3D-static-spheroid EV was 182.5 nm and the mode diameter was 106.1 nm.

### Example 6. Analysis of expression markers of extracellular vesicles derived from 3D spheroid-type cell aggregate

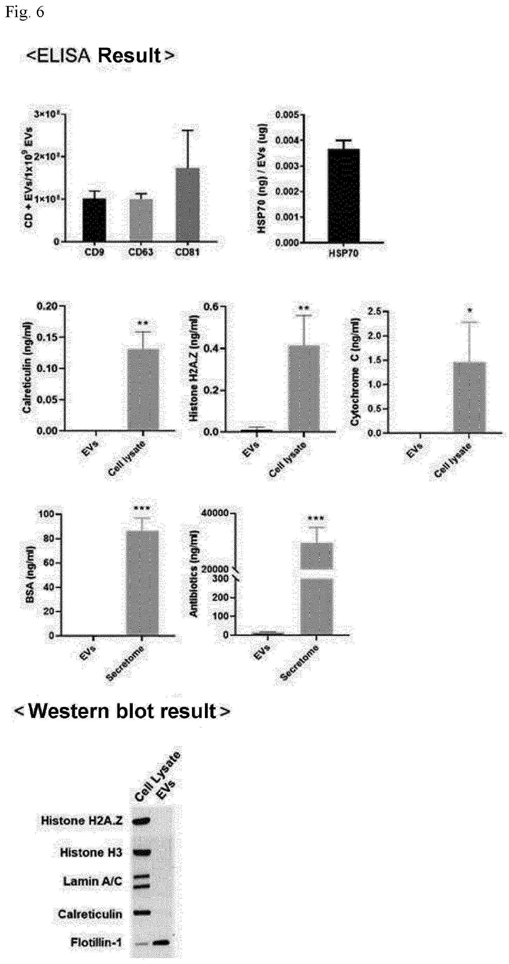

Experiments were performed to confirm expression markers of 3D-static-spheroid EVs isolated in Example 1.3. A cell lysate and a secretome were used as a control. The cell lysate was prepared by a method of washing 3D-static-spheroids with PBS, treating the 3D-static-spheroids with trypsin, recovering cells, and obtaining a cell pellet using a centrifuge of the recovered cells. The secretome was prepared by a method of obtaining the cell pellet, isolating EVs from a supernatant of the culture medium through the process of Example 1.3, and obtaining the remaining culture secretome. For marker analysis, extracellular vesicles-specific positive markers CD9, CD63, CD81 and HSP70 were quantified using ELISA, and specific contaminant protein markers such as calreticulin, histone H2A.Z, cytochrome C, albumin, and antibiotics were quantified. In addition, histone H2A.Z, histone H3, lamin A/C, and calreticulin, which were specific contaminant protein markers of extracellular vesicles, were quantified using Western blot, and a positive marker of extracellular vesicles, flotillin-1 was quantified. The ELISA analysis result and the Western blot result were shown in FIG. 6.

As illustrated in FIG. 6, it was confirmed that 3D-static-spheroid EVs expressed all of the extracellular vesicles-specific positive markers CD9, CD63, CD81 and HSP70, and calreticulin, histone H2A.Z, histone H3, cytochrome C, albumin (BSA, bovine serum albumin), lamin A/C, and antibiotics, which were highly expressed contaminant protein markers in the cell lysate and the secretome, were not almost expressed. In particular, it was confirmed that flotillin-1, a positive marker for extracellular vesicles, which was expressed very low in the cell lysate, was relatively highly expressed in 3D-static-spheroid EVs.

### Example 7. Analysis of production of extracellular vesicles derived from 3D spheroid-type cell aggregate

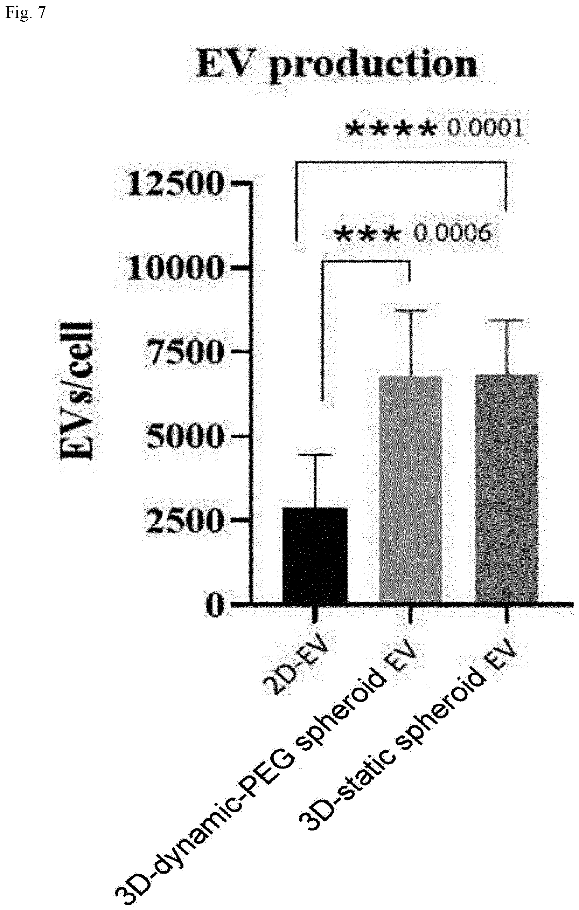

The experiment was performed to compare the productions of the 3D-static-spheroid EVs, which were the extracellular vesicles produced by the production method of Example 1, extracellular vesicles (3D-dynamic-PEG spheroid EVs) isolated from the 3D-dynamic-PEG spheroids of Example 2, and extracellular vesicles (2D-EVs) isolated from stem cells cultured by a conventional 2D culture method. The 2D-EVs were prepared by the following process. The stem cells cultured for 3 days in a cell stack were washed with PBS, replaced with a serum-free medium, and further cultured for 2 days. The culture medium was recovered and the cell debris was continuously removed using centrifugation and a 0.2 µm filter. Thereafter, the culture medium passed through a hollow fiber membrane using a TFF system to remove proteins, and the EVs were first isolated, and purified once more with physiological saline to obtain high-purity 2D-EVs. The productions per cell derived from the prepared 3D-static-spheroid EVs, 3D-dynamic-PEG spheroid EVs, and 2D-EVs were compared and illustrated in Table 2 and FIG. 7.

**[Table 2]**

| | 2D-EV | 3D-dynamic-PEG spheroid EV | 3D-static-spheroid EV |
|---|---|---|---|
| Production (EVs/cell) | 2437 EVs/cell | 6791 EVs/cell | 6840 EVs/cell |

### Example 8. Expression microRNA (miRNA) Analysis of extracellular vesicles derived from 3D spheroid-type cell aggregate

### 8.1 Identification of miRNAs with increased expression in 3D-static-spheroid EVs

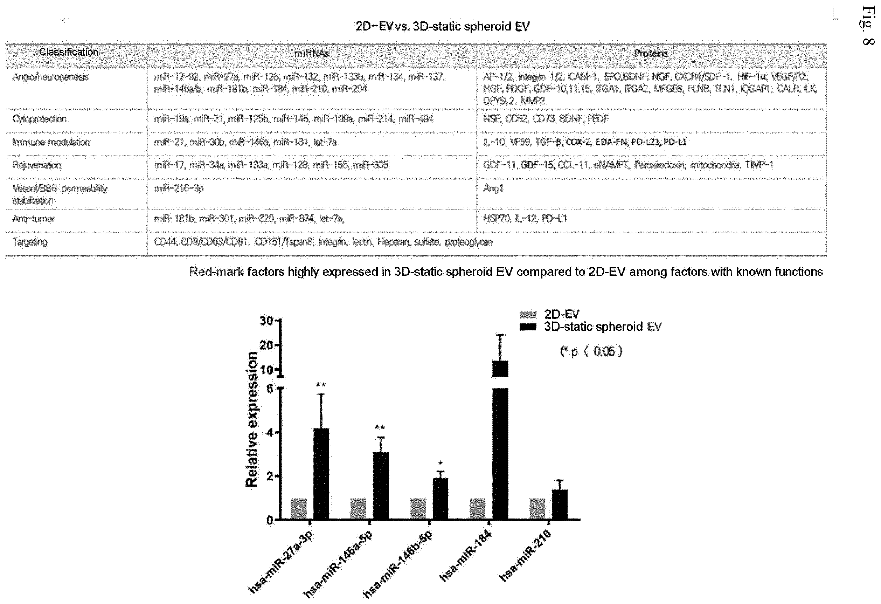

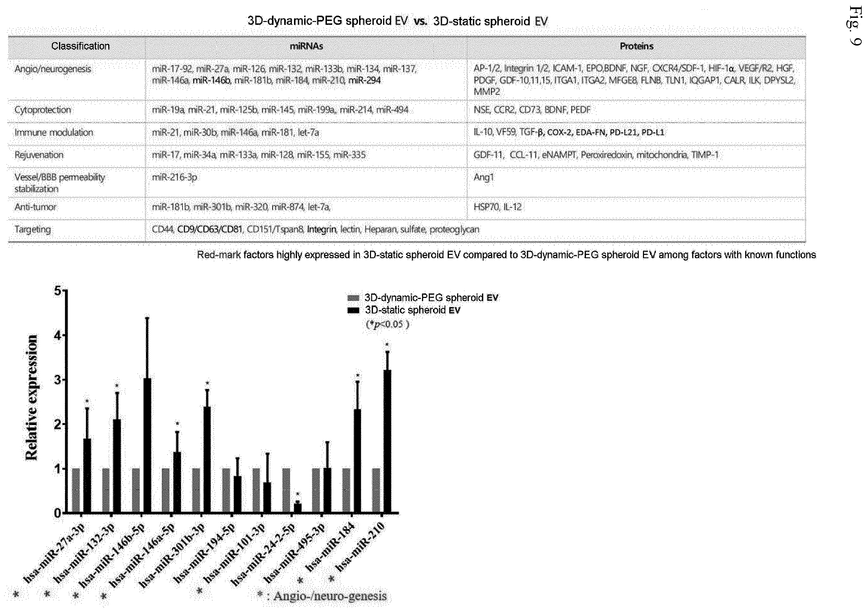

In order to confirm the differences in characteristics between 3D-static-spheroid EVs which were the extracellular vesicles produced by the production method of Example 1, 3D-dynamic-PEG spheroid EVs as extracellular vesicles isolated from 3D-dynamic-PEG spheroids of Example 2, and 2D-EVs prepared in Example 7, expressions of miRNAs and protein expressions were measured by qPCR. miRNAs and proteins highly expressed in the 3D-static-spheroid EVs compared to the 2D-EVs were illustrated in FIG. 8, and miRNAs and proteins highly expressed in the 3D-static-spheroid EVs compared to the 3D-dynamic-PEG spheroid EVs were illustrated in FIG. 9.

As illustrated in FIG. 8, it was confirmed that as compared with the 2D-EVs, the 3D-static-spheroid EVs highly expressed miR-27a, miR-146b, and miR-146a as miRNAs that exhibited the efficacy on angio/neurogenesis and immune regulation, and highly expressed integrin 1/2 and Vascular endothelial growth factor/R2 (VEGF/R2) as proteins exhibiting the efficacy on angio/neurogenesis.

As illustrated in FIG. 9, it was confirmed that as compared with the 3D-dynamic-PEG spheroid EVs, the 3D-static-spheroid EVs highly expressed miR-146a as miRNA exhibiting the efficacy on angio/neurogenesis and immune regulation, miR-27a, miR-132, miR-184 and miR-210, which were miRNAs exhibiting the efficacy on angio/neurogenesis, and miR-301b exhibiting antitumor efficacy, and highly expressed integrin 1/2 and VEGF/R2 as proteins exhibiting the efficacy on angio/neurogenesis.

From these results, it was confirmed that as compared to the 2D-EVs or 3D-dynamic-PEG spheroid EVs, the 3D-static-spheroid EVs, which were the extracellular vesicles produced by the production method of Example 1, were new extracellular vesicles with different miRNA and protein expression, and particularly, extracellular vesicles which may be clinically useful by highly expressing miRNAs related to angio/neurogenesis, immune regulation, rejuvenation or antitumor.

### 8.2 Analysis of miRNA expression difference according to donor

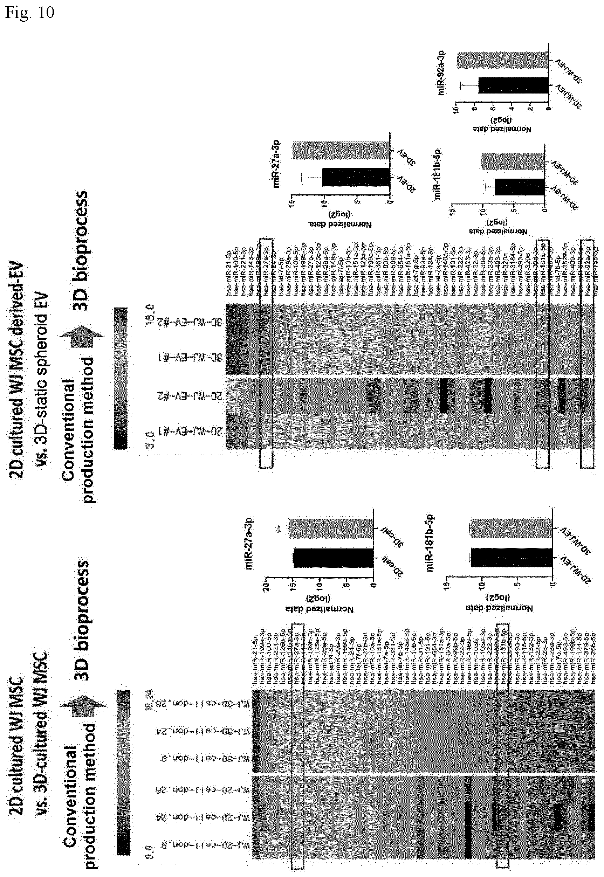

Stem cell therapy was known to have a problem of donor variation in which components varied depending on a donor. An experiment was performed to confirm whether the 3D-static spheroid EVs of the present invention exhibited a more consistent miRNA profile without a donor variation issue. Samples from each donor were received from the Samsung medical center (Seoul, Korea) and used. Donor-specific miRNA profiles were compared with each other in 2D-cultured WJ-MSCs and 3D-cultured WJ-MSCs of Example 1.1 and 3D-static-spheroid EVs, which were the extracellular vesicles produced by the production method of Example 1. miRNA was profiled by a Small RNA sequencing method, and the results were shown in FIG. 10.

As illustrated in FIG. 10, it was confirmed that 2D-cultured WJ-MSCs and 2D-EVs had a large difference in miRNA composition produced depending on the donor, whereas 3D-cultured WJ-MSCs and 3D-static-spheroid EVs had a decreased difference depending on the donor. In particular, it was confirmed that the 3D-static-spheroid EVs showed a consistent miRNA profile without a difference depending on the donor, and at the same time, particularly, as compared with 2D-EVs, miR-27a-3p (1.5-fold), miR-146a-5p (2.3-fold), miR-210 (2.6-fold), and miR-132 (2.6-fold), which were miRNAs capable of exhibiting the angiogenic or nerve regenerative effects, were more uniformly included. In addition, it was confirmed that miR-199a, miR-125b, miR-26a, let-7, miR-125a, miR-181b, and miR-92a, which were miRNAs related to angiogenesis or nerve regeneration, were expressed at uniform levels among respective donors.

These results indicated that the 3D-static-spheroid EVs may reduce the difference in therapeutic active ingredients according to a donor and exhibit better therapeutic effects.

### Example 9. Analysis of expression mRNA when treating cells with extracellular vesicles derived from 3D spheroid-type cell aggregate

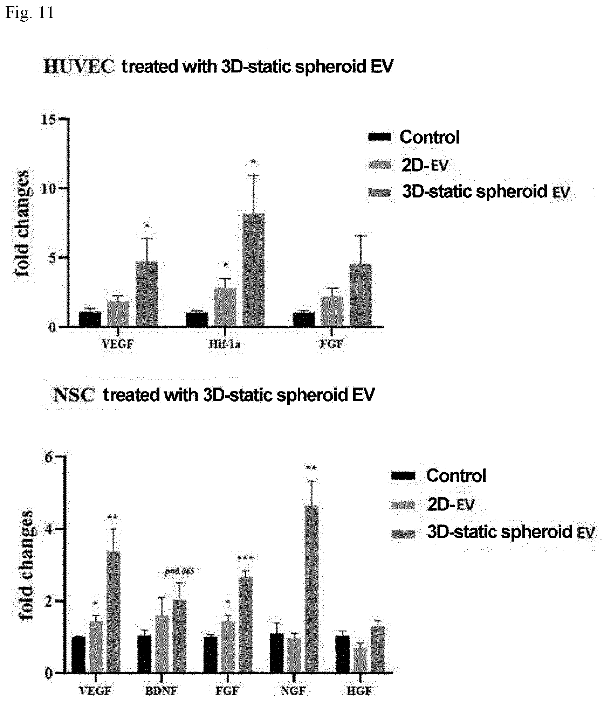

Human umbilical vascular endothelial cells (HUVECs) or primitive neural stem cells (pNSCs) were treated with 3D-static-spheroid EVs, which were the extracellular vesicles produced by the production method of Example 1, and 2D-EVs, which were extracellular vesicles isolated from stem cells cultured by a conventional 2D culture method, and then changes in mRNA expression were confirmed through qPCR, and the qPCR results were shown in FIG. 11.

As illustrated in FIG. 11, compared to cells treated with 2D-EVS, the HUVECs treated with the 3D-static-spheroid EVs increased the expression of a vascular endothelial growth factor (VEGF), a hypoxia-inducible factor 1-alpha (Hif-1a), and a fibroblast growth factor (FGF), and the pNSCs treated with the 3D-static-spheroid EVs increased the expression of VEGF, a brain-derived neurotrophic factor (BDNF), FGF, a nerve growth factor (NGF), and a hepatocyte growth factor (HGF). In particular, it was confirmed that in the case of pNSCs treated with the 2D-EVs compared to a control group, the expression of NGF and HGF decreased, but pNSCs treated with the 3D-static-spheroid EVs showed an effect of increasing the expression of NGF and HGF, and thus the 3D-static-spheroid EVs and 2D-EVs exhibited qualitatively different effects.

The VEGF, BDNF, NGF, HGF and Hif-1a were angio/neurogenesis-related proteins, and the FGF was a factor corresponding to a protein that regulated biological functions related to cell proliferation, survival, and differentiation. Thus, it was confirmed that 3D-static-spheroid EVs with relatively high increased expression effects of VEGF, BDNF, NGF, HGF, Hif-1a and FGF were excellent extracellular vesicles for clinical application.

### Example 10. Effect of 3D-static-spheroid EV on stroke rat model

### 10.1 Preparation of stroke rat model

To prepare a stroke rat model, transient middle cerebral artery occlusion (tMCAo) was induced using an intraluminal vascular occlusion method. Specifically, male Sprague-Dawley rats (8 weeks, 270 to 300 g) were used, anesthetized with 1.5% isoflurane, 70% N₂O and 30% O₂, and the body temperature was maintained at 37.0 to 37.5°C using a heating pad during the surgical time. A 4-0 surgical single filament nylon suture with a rounded tip was advanced into the lumen of the internal carotid artery until occluding the origin of the middle cerebral artery in the left common carotid artery. The transient middle cerebral artery occlusion (tMCAo) was performed by withdrawing the suture until the tip occluded the lumen of the common carotid artery, and reperfusion was performed after 90 minutes. Rats with hemorrhagic transformation or subarachnoid hemorrhage induced by the rupture of intracranial arteries and rats that did not show observable neurological deficits after the transient middle cerebral artery occlusion (tMCAo) were excluded from the analysis.

### 10.2 Verification of brain infarct area reduction effect of 3D-static-spheroid EV in stroke rat model

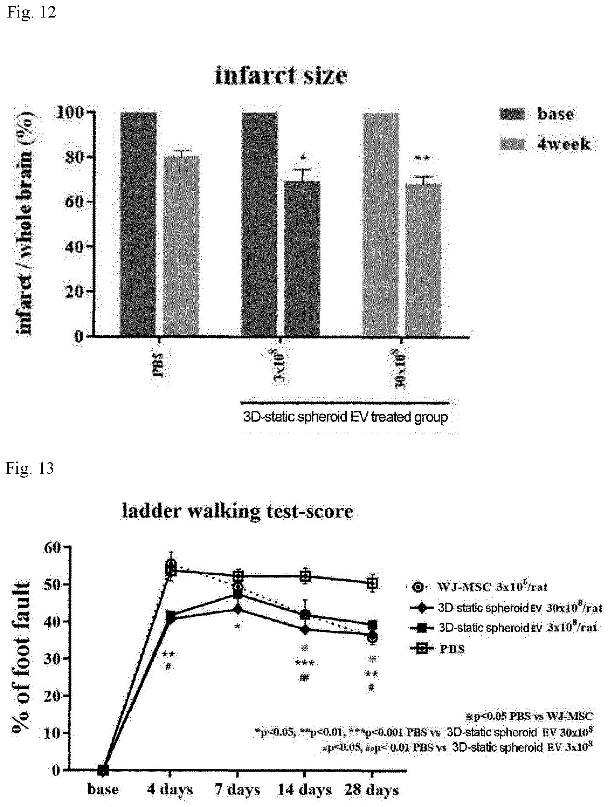

For the stroke rat model prepared in Example 10.1, the treatment concentrations of 3D-static-spheroid EVs were 3 × 10⁸ and 30 × 10⁸ EVs/rat, respectively, and after 4 weeks of the treatment, MRI was taken to measure changes in the infarct area. As a control group, a PBS-treated group was used, and the measured changes in the infarct area were shown in FIG. 12.

As illustrated in FIG. 12, it was confirmed that the infarct area was significantly reduced in all experimental groups treated at a concentration of 3 × 10⁸ EVs/rat or higher than the control group.

As such a result, it was confirmed that considering that extracellular vesicles derived from a spheroid-type cell aggregate prepared by a conventional known 3D method showed a significant degree of brain infarct area reduction effect when treated at a concentration of 30 × 10⁸ EVs/rat or higher, 3D-static-spheroid EVs showed the effect of effectively reducing the brain infarct area even with a small amount.

### 10.3 Verification of neurological behavioral recovery ability of 3D-static-spheroid EV on stroke rat model

For the stroke rat model prepared in Example 10.1, the treatment concentrations of 3D-static-spheroid EVs were 3 × 10⁸ and 30 × 10⁸ EVs/rat, respectively, and a ladder walking test was performed to verify the neurological behavioral recovery ability of 3D-static-spheroid EVs. A PBS-treated group was used as a control group, and human umbilical cord-derived mesenchymal stem cells (WJ-MSC) at a concentration of 3 × 10⁸ cells/rat were used as a comparative group.

Specifically, the ladder walking test method was as follows. The ladder walking test was performed by a single observer unknowing experimental condition before the transient middle cerebral artery occlusion (tMCAo) and on 4, 7, 14 and 28 days after the transient middle cerebral artery occlusion. A ladder apparatus for the experiment consisted of a metal horizontal ladder across which the animal walked and a transparent sidewall. The number of trips (foot fault) and the total number of steps were measured to calculate a ratio % of foot fault, and the calculated ratio % of foot fault was illustrated in FIG. 13.

As illustrated in FIG. 13, as the result of the ladder walking test measured after 4 days of the middle cerebral artery occlusion, it was confirmed that the ratio % of foot fault was significantly reduced compared to the control group only in the 3D-static-spheroid EV treated group at concentrations of 3 × 10⁸ and 30 × 10⁸ EVs/rat, and particularly, it was confirmed that the 3D-static-spheroid EV-treated group at a concentration of 30 × 10⁸ EVs/rat reduced the ratio % of foot fault in all sections.

From these results, compared to the mesenchymal stem cells, it was confirmed that the 3D-static-spheroid EVs rapidly induced the neurological behavior recovery. As such a result, it was shown that the 3D-static-spheroid EVs of the present invention effectively induced the neurological behavior recovery even in a small amount, as compared with the fact the extracellular vesicles derived from a spheroid-type cell aggregate prepared by a conventional known 3D method were treated at a concentration of 30 × 10⁸ EVs/rat or higher to exhibit the significant neurological behavior recovery effect.

### Example 11. Evaluation of cell incorporation ability and miRNA delivery ability of 3D-static-spheroid EVs

### 11.1 Evaluation of cell incorporation ability of 3D-static-spheroid EVs

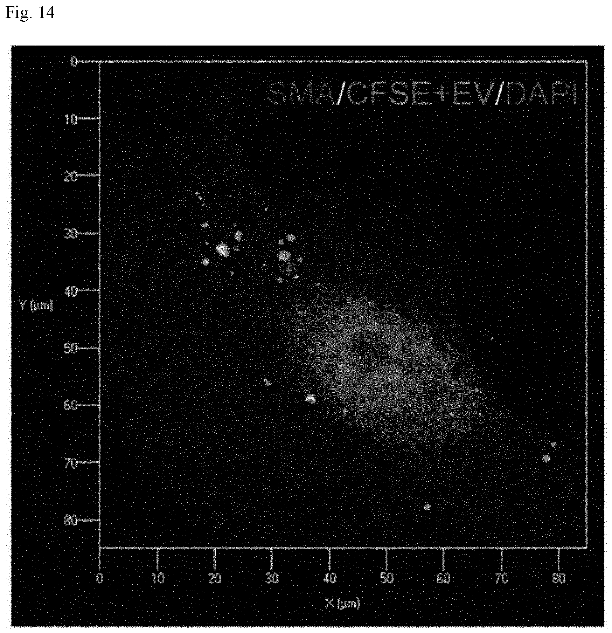

To evaluate the cell incorporation ability of 3D-static-spheroid EVs, a human NSC cell line (ReNcells^{®}) was treated with CFSE-labeled 3D-static-spheroid EVs and cultured for 24 hours, and confirmed with a confocal microscope (LSM 700, Carl Zeiss, Germany), which was illustrated in FIG. 14.

As illustrated in FIG. 14, when cells were treated with the 3D-static-spheroid EVs, it was confirmed that the 3D-static-spheroid EVs were incorporated into cells and internalized.

### 11.2 Evaluation of miRNA expression by treatment of 3D-static-spheroid EVs

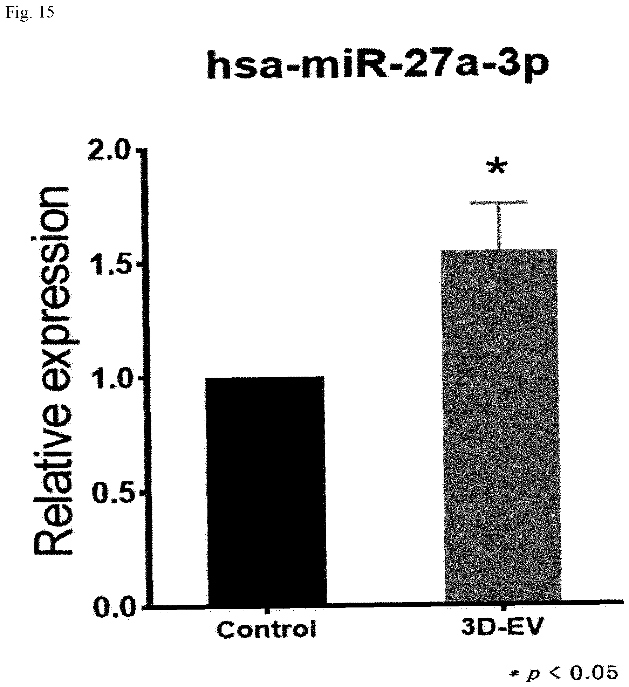

The expression of miR-27a-3p was measured using RT-qPCR in the human NSC cell line (ReNcells^{®}) treated with CFSE-labeled 3D-static-spheroid EVs in Example 11.1. As a control group, a human NSC cell line (ReNcells^{®}) untreated with the labeled 3D-static-spheroid EVs was used, and the results were shown in FIG. 15.

As illustrated in FIG. 15, it was confirmed that the expression of miR-27a-3p significantly increased in cells treated with the 3D-static-spheroid EVs compared to the control group.

Through this, it was confirmed that when the cells were treated with the 3D-static-spheroid EVs, a clinically significant substance highly expressed in the 3D-static-spheroid EVs, such as miR-27a-3p, was effectively delivered to the cells.

As described above, specific parts of the present invention have been described in detail, and it will be apparent to those skilled in the art that these specific techniques are merely preferred embodiments, and the scope of the present invention is not limited thereto. Therefore, the substantial scope of the present invention will be defined by the appended claims and their equivalents.

## Claims

1. A method for producing extracellular vesicles derived from a three-dimensional spheroid-type cell aggregate comprising:
(a) preparing the three-dimensional spheroid-type cell aggregate by 3 dimensionally (3D)-culturing stem cells in a microwell with a diameter of 200 to 800 µm and a depth of 100 to 1000 µm; and
(b) isolating the extracellular vesicles from the three-dimensional spheroid-type cell aggregate.

2. The method for producing the extracellular vesicles derived from the three-dimensional spheroid-type cell aggregate of claim 1, wherein the stem cells are at least one selected from the group consisting of mesenchymal stem cells, pluripotent stem cells, induced pluripotent stem cells and embryonic stem cells.

3. The method for producing the extracellular vesicles derived from the three-dimensional spheroid-type cell aggregate of claim 1, wherein the 3D culture in step (a) is static culture.

4. The method for producing the extracellular vesicles derived from the three-dimensional spheroid-type cell aggregate of claim 1, wherein the 3D culture in step (a) is performed for 1 to 10 days.

5. The method for producing the extracellular vesicles derived from the three-dimensional spheroid-type cell aggregate of claim 1, wherein the 3D culture in step (a) is performed by dispersing and culturing the mesenchymal stem cells in the microwell at a density of 100 to 1000 cells/well.

6. The method for producing the extracellular vesicles derived from the three-dimensional spheroid-type cell aggregate of claim 1, wherein the isolating of the extracellular vesicles in step (b) is performed by physical isolation or chemical isolation.

7. The method for producing the extracellular vesicles derived from the three-dimensional spheroid-type cell aggregate of claim 1, further comprising:
filtering the extracellular vesicles with a filter after centrifuging before the isolating of the extracellular vesicles in step (b).

8. The method for producing the extracellular vesicles derived from the three-dimensional spheroid-type cell aggregate of claim 1, wherein the isolating of the extracellular vesicles in step (b) is performed by a tangential flow filtration (TFF) method.

9. The method for producing the extracellular vesicles derived from the three-dimensional spheroid-type cell aggregate of claim 1, wherein the extracellular vesicles highly express at least one selected from the group consisting of miR-146a, miR-27a, miR-132, miR-184, miR-210 and miR-301b, as compared with extracellular vesicles derived from a spheroid-type cell aggregate obtained by 3D-dynamic culture of mesenchymal stem cells.

10. The method for producing the extracellular vesicles derived from the three-dimensional spheroid-type cell aggregate of claim 1, wherein the extracellular vesicles highly express at least one selected from the group consisting of miR-27a, miR-146a and miR-146b, as compared with extracellular vesicles derived from 2D-cultured mesenchymal stem cells.

11. The method for producing the extracellular vesicles derived from the three-dimensional spheroid-type cell aggregate of claim 1, wherein the extracellular vesicles highly express at least one selected from the group consisting of Integrin 1/2 and Vascular Endothelial Growth Factor Receptor 2 (VEGF/R2), as compared with extracellular vesicles derived from a spheroid-type cell aggregate obtained by 3D-dynamic culture of mesenchymal stem cells.

12. The method for producing the extracellular vesicles derived from the three-dimensional spheroid-type cell aggregate of claim 1, wherein the extracellular vesicles highly express at least one selected from the group consisting of Integrin 1/2 and Vascular Endothelial Growth Factor Receptor 2 (VEGF/R2), as compared with extracellular vesicles derived from 2D-cultured mesenchymal stem cells.

13. The method for producing the extracellular vesicles derived from the three-dimensional spheroid-type cell aggregate of claim 1, wherein the three-dimensional spheroid-type cell aggregate has an average diameter of 65 to 133 µm.

14. The method for producing the extracellular vesicles derived from the three-dimensional spheroid-type cell aggregate of claim 1, wherein the microwell is coated with at least one selected from the group consisting of TMSPMA (3-(Trimetoxysily) propylmethacrylate), HEA (Hydroxyethyl acrylate), GMA (Glycidyl methacrylate), EGDMA (diethyleneglycol dimethacrylate), THFA (Tetrahydrofurfuryl acrylate), HMAA (Hydroxymethul acrylamide), PEA (Phenyl epoxyacrylate), HOFHA (6-Hydroxy-2, 2,3,3,4,4,5,5-octafluoro), EOPT (Polyethoxylated(4)pentaerythritoltetraacrylate), HPA (Hydroxypropyl acrylate), BMA (Buthylmethacrlate), PETIA (Pentaerythritol triacrylate), HDDA (Hexan diol diacrylate), EGPEA (Ethyleneglycol phenyletheracrylate), BM (Benzylmethacrylate), HPPA (Hydroxyphenoxypropyl acrylate), BHPEA (2-(4-Benzoyl-3-hydroxyphenoxy)ethylacrylate), HEMA (Hydroxyethyl methacrylate), HPMA (N-(2-Hydroxypropyl) methacrylamide) and MPC (2-Methacryloyloxyethyl Phosphorylcholine Polymer).

15. The method for producing the extracellular vesicles derived from the three-dimensional spheroid-type cell aggregate of claim 1, wherein the extracellular vesicles derived from the three-dimensional spheroid-type cell aggregate are reduced in donor variation.

16. Extracellular vesicles derived from a three-dimensional spheroid-type cell aggregate produced by the production method of any one of claims 1 to 15.

17. The extracellular vesicles derived from the three-dimensional spheroid-type cell aggregate of claim 16, wherein the extracellular vesicles derived from the three-dimensional spheroid-type cell aggregate are reduced in donor variation.

18. A composition for use in producing extracellular vesicles comprising a three-dimensional spheroid-type cell aggregate produced by 3 dimensionally (3D)-culturing mesenchymal stem cells in a microwell with a diameter of 200 to 800 µm and a depth of 100 to 1000 µm.

19. A method for reducing a donor variation of extracellular vesicles derived from a cell aggregate comprising:
(a) preparing a three-dimensional spheroid-type cell aggregate by 3 dimensionally (3D)-culturing stem cells in a microwell with a diameter of 200 to 800 µm and a depth of 100 to 1000 µm; and
(b) isolating the extracellular vesicles from the three-dimensional spheroid-type cell aggregate.

20. A method for producing extracellular vesicles derived from a cell aggregate with a reduced donor variation comprising:
(a) preparing a three-dimensional spheroid-type cell aggregate by 3 dimensionally (3D)-culturing stem cells in a microwell with a diameter of 200 to 800 µm and a depth of 100 to 1000 µm; and
(b) isolating the extracellular vesicles from the three-dimensional spheroid-type cell aggregate.
